Europäisches Patentamt

European Patent Office

Office européen des brevets

(11) Veröffentlichungsnummer : **0 142 784**
**B1**

## (12) EUROPÄISCHE PATENTSCHRIFT

(45) Veröffentlichungstag der Patentschrift :
20.01.88

(21) Anmeldenummer : 84113506.4

(22) Anmeldetag : 08.11.84

(51) Int. Cl.⁴ : **C 09 C 3/10, C 08 K 9/10, C 08 F285/00, C 08 F292/00// A61K6/08**

(54) Anorganisch-organische Füllstoffe, Verfahren zu deren Herstellung sowie deren Verwendung in polymerisierbaren Massen.

(30) Priorität : 19.11.83 DE 3341888

(43) Veröffentlichungstag der Anmeldung :
29.05.85 Patentblatt 85/22

(45) Bekanntmachung des Hinweises auf die Patenterteilung : 20.01.88 Patentblatt 88/03

(84) Benannte Vertragsstaaten :
AT BE CH DE FR GB IT LI NL SE

(56) Entgegenhaltungen :
DE-A- 1 937 871
DE-A- 2 405 578
RESEARCH DISCLOSURE, Nr. 162, Oktober 1977, (GB),Seite 80, Spalte Nr. 16269 "New dental materials".
Die Akte enthält technische Angaben, die nach dem Eingang der Anmeldung eingereicht wurden und die nicht in dieser Patentschrift enthalten sind.

(73) Patentinhaber : BAYER AG
Konzernverwaltung RP Patentabteilung
D-5090 Leverkusen 1 Bayerwerk (DE)

(72) Erfinder : Podszun, Wolfgang, Dr.
Wolfskaul 4
D-5000 Köln 80 (DE)

**Beschreibung**

Gegenstand der Erfindung ist ein Füllstoff für polymerisierbare Massen, insbesondere Dentalwerkstoffe, mit einem anorganischen Kern aus Siliziumdioxid, Silikaten oder Aluminiumoxid, der eine Teilchengröße von 10 bis 500 nm aufweist, und einer Hülle aus polymerisiertem Vinylsilan, wobei der Anteil des Vinylsilans 2 bis 40 Gew.-%, bezogen auf den anorganischen Kern, beträgt.

Mit anorganischen Füllstoffen gefüllte Polymersysteme finden in Dentalwerkstoffen breite Anwendung. So werden als Zahnfüllungsmaterialien auf Kunststoffbasis im allgemeinen härtbare Gemische aus (Meth)acrylsäureestern als polymerisierbare Bindemittel und feinteilige anorganische Füllstoffe eingesetzt.

Nach ihrer Teilchengröße lassen sich diese Füllstoffe in « Makrofiller » mit einer Teilchengröße von ca. 1 μm bis ca. 50 μm (vorzugsweise ca. 30 μm) und in « Mikrofiller » mit einer Teilchengröße von ca. 5-500 nm unterteilen. Dentalwerkstoffe auf Basis von « Makrofillern » werden beispielsweise in US-A-3066112, US-A-3926906, DE-A 2357324 und DE-A 2419887 beschrieben.

In DE-A 2403211 und DE-A 2462271 werden Werkstoffe für Dentalzwecke offenbart, die als anorganischen Füllstoff ausschließlich mikrofeines Siliziumdioxid mit einer Teilchengröße von 5 bis 700 nm enthalten. Die « Mikrofillerprodukte » zeigen gegenüber den « Makrofillerprodukten » eine deutlich verbesserte Polierbarkeit, weisen aber im allgemeinen einen geringeren Füllgrad auf, was sich in einigen physikalischen Eigenschaften wie Polymerisationsschrumpf, thermischem Ausdehnungskoeffizienten und Wasseraufnahme ungünstig auswirkt. Darüber hinaus weisen Formulierungen aus « Mikrofillern » und polymerisierbaren Bindemitteln schlechte Verarbeitungseigenschaften aufgrund großer Klebrigkeit auf. Man hat verschiedene Wege beschritten, um den Schwierigkeiten des Einarbeitens von « Mikrofillern » in monomere Bindemittel zu begegnen. So ist es möglich, die « Mikrofillerpartikel » ganz oder teilweise durch « Mikrofiller »-haltige Vorpolymerisate in Splitterform zu ersetzen. Das Einbringen der « Mikrofillerpartikel » in Perlpolymerisate stellt eine weitere Verbesserung dar (EP-A 01190). Die Verwendung von füllstoffhaltigen Vorpolymerisaten in Splitter- oder Perlform führt zu « Mikrofillerprodukten » mit einem Füllstoffgehalt von ca. 50 Gew.-% als Obergrenze. Höhere Füllgrade können erreicht werden, indem die « Mikrofiller » vor dem Einsatz agglomeriert und zu Granulaten aufgemahlen werden, wobei man jedoch Einbußen hinsichtlich Homogenität und Polierbarkeit des Dentalwerkstoffes in Kauf nehmen muß (PCT/EP 80/00135).

Die Beschichtung von « Makrofillern » mit organischen Polymeren zum Zwecke einer verbesserten Einarbeitbarkeit in Dentalwerkstoffe ist eine bekannte Methode. So wird in der DE-A 19 37 871 ein feinverteiltes Siliziumdioxid- oder Silikat-Füllmittel, das mit einem Vinylsilan als Grundierung und mit einem Acrylpolymer überzogen ist, beschrieben. Die EP-A 0 047 971 offenbart ebenfalls mineralische Teilchen, die mit Kunststoff beschichtet sind. Aus der JA-A 74-042905 ist ein mit synthetischen Polymeren beschichtetes Glasgranulat als Füllstoff für Zahnfüllmassen bekannt.

In der DE-A 24 05 578 werden silanisierte Mikrofüller für Dentalwerkstoffe beschrieben. Bei der Anwendung weisen sie eine Klebrigkeit auf, die die Anwendung erschwert.

Diese bekannten Beschichtungsverfahren können jedoch nicht in der beschriebenen Weise auf « Mikrofiller » übertragen werden, da « Mikrofiller » wegen ihrer äußerst geringen Teilchengröße und hohen spezifischen Oberfläche bei der Nachbehandlung zur Bildung von Agglomeraten neigen, wobei die vorteilhaften Eigenschaften der « Mikrofiller » verloren gehen.

Aufgabe der Erfindung ist es, Dentalwerkstoffe zu schaffen, die nicht die schlechte Verarbeitbarkeit der bekannten Mikrofüller mit nur einer Silanhülle und die geringe Verschleißfestigkeit der bekannten Makrofüller mit Silanhülle und (Meth)acrylathülle aufweisen.

Es wurden Füllstoffe für polymerisierbare Massen mit einem anorganischen Kern aus Siliziumdioxid, Silikaten oder Aluminiumoxid, der eine Teilchengröße von 10 bis 500 nm aufweist, und einer Hülle aus polymerem Vinylsilan, wobei der Anteil des Vinylsilans 2 bis 40 Gew.-%, bezogen auf den anorganischen Kern, beträgt, gefunden, die dadurch gekennzeichnet sind, daß der Füllstoff eine zweite Hülle aus (Meth)acrylatpolymeren aufweist.

Mit den erfindungsgemäßen Füllstoffen wird überraschenderweise die bei den bekannten Mikrofillern auftretende Klebrigkeit vermieden oder herabgesetzt, wobei die sonstigen erwünschten positiven Eigenschaften, wie gute Polierbarkeit und hohe mechanische Festigkeit, erhalten bleiben.

Es wurde auch ein Verfahren zur Herstellung eines Füllstoffes für polymerisierbare Massen mit einem anorganischen Kern mit einer Teilchengröße von 10 bis 500 nm, einer ersten Hülle aus Vinylsilan, wobei der Anteil des Vinylsilans 2 bis 40 Gew.-%, bezogen auf den anorganischen Kern, beträgt, und einer zweiten Hülle aus (Meth)acrylatpolymeren gefunden, das dadurch gekennzeichnet ist, daß man für die erste Hülle zuerst die Silanverbindung durch Umsetzung mit etwa gleichen Gewichtsteilen Wasser in Gegenwart von 0,1 bis 5 Gew.-% einer Säure in eine aktivierte Form überführt, dann den anorganischen Kern in einer Konzentration von 5 bis 40 Gew.-% in einem organischen Lösungsmittel dispergiert und im Temperaturbereich von 0 bis 100 °C mit dem aktiven Silan-Wasser-Gemisch versetzt, und dann für die zweite Hülle den silanisierten anorganischen Kern in einem inerten organischen Lösungsmittel mit (Meth)acrylatmonomeren und mit einem Polymerisersationsstarter versetzt und die Polymerisationsreaktion durchführt.

Als hochdisperser Füllstoff sind alle konventionellen anorganischen Füllstoffe mit Teilchengrößen von 10-500 nm, insbesondere 10-100 nm, vorzugsweise auf Basis von Aluminiumoxid, Siliziumdioxid und Silikaten, geeignet. Besonders bevorzugt werden flammhydrolytisch gewonnenes Siliziumdioxid und Aluminiumdioxid mit einer Teilchengröße von 10 bis 40 nm und einer BET-Oberfläche von 30 bis 300 m²/g, vorzugsweise 40 bis 200 m²/g.

Für die erste Hülle aus Vinylsilan können z. B. die aus den eingangs erwähnten Druckschriften an sich bekannten Vinylsilane, wie zum Beispiel Vinyltriethoxysilan, Vinyltrimethoxysilan, γ-Methacryloxypropyltrimethoxysilan, γ-Methacryloxypropyl-tri(2-methoxy)silan oder Vinyltriacetoxysilan eingesetzt werden. γ-Methacryloxypropyltrimethoxisilan wird bevorzugt eingesetzt. Die Menge an Silanverbindung beträgt dabei bevorzugt 2-40 Gew.-%, besonders bevorzugt 5-25 Gew.-%, bezogen auf den hochdispersen Füllstoff.

Zur Herstellung der erfindungsgemäßen Füllstoffe ist es notwendig, definierte Silanisierungsbedingungen einzuhalten. Die Silanverbindung wird in einer ersten Reaktionsstufe durch Reaktion mit Wasser in eine aktivierte Form überführt. Bei dieser Vorreaktion werden vermutlich die weniger reaktiven Alkoxy- bzw. Acetoxy-Gruppen der Silanverbindung zu den reaktiveren Silanolgruppen hydrolysiert. Diese reaktive Mischung ist nicht lagerstabil und muß jeweils vor der Silanisierungsreaktion frisch zubereitet werden. Die notwendige Reaktionszeit der Silan-Wasservorreaktion hängt von der Reaktionstemperatur ab und liegt bei Raumtemperatur in der Regel im Bereich von 15 Min. bis 2 Stunden. Besonders gute Ergebnisse werden erzielt, wenn Wasser und Silanverbindung in etwa gleichen Gewichtsanteilen eingesetzt werden. Die günstigste Reaktivität der Silanverbindung liegt zu dem Zeitpunkt vor, an dem das zunächst zweiphasige Wasser-Silangemisch gerade einphasig geworden ist. Als Katalysator können sowohl Mineralsäuren als auch organische Säuren verwendet werden. Gut geeignet sind z. B. Acrylsäure und Methylacrylsäure. Die Säure wird vorzugsweise in Mengen von 0,1 bis 5 Gew.-%, besonders bevorzugt 0,5 bis 2 Gew.-%, bezogen auf die Silanverbindung, eingesetzt.

Die Silanisierung erfolgt vorzugsweise in Gegenwart eines inerten organischen Lösungsmittels, wie z. B. Aceton, Essigester, Chloroform oder Methylenchlorid. Der hochdisperse Füllstoff wird vorzugsweise in einer Konzentration von 5-40 Gew.-%, besonders bevorzugt 15. 25 Gew.-% im organischen Lösungsmittel mit Hilfe eines hochtourigen Rührers dispergiert und mit dem vorbereiteten aktiven Silan-Wasser-Gemisch versetzt. Die Silanisierungsreaktion kann zwischen 0 und 100 °C, z. B. bei Raumtemperatur, vorteilhafterweise jedoch bei erhöhter Temperatur. z. B. bei der Siedetemperatur des Lösungsmittels, durchgeführt werden.

Die Reaktionszeit beträgt im allgemeinen einige Stunden. Während der Silanisierung nimmt die Viskosität der Dispersion ab, so daß das Ende der Reaktion in einfacher Weise durch laufende Messung der Viskosität bestimmt werden kann.

Zum Aufbau der 2. Hülle wird die Dispersion des silanisierten Füllstoffs in einem inerten organischen Lösungsmittel mit (Meth)acrylatmonomeren und mit einem Polymerisationsstarter versetzt und die Polymerisationsreaktion eingeleitet. Die Zugabe der Monomeren kann in einer Portion auf einmal zu Beginn der Reaktion erfolgen. Es ist jedoch günstiger, die Monomeren und den Polymerisationsstarter in das Reaktionsgemisch portionsweise über einen längeren Zeitraum hinweg zu dosieren.

Als (Meth)acrylatmonomere sind sowohl monofunktionelle (Meth)acrylate als auch Di- und Tri-(meth)acrylate geeignet. In einer besonderen Ausführungsform der vorliegenden Erfindung werden zumindest anteilmäßig Di-bzw. Tri(meth)acrylate eingesetzt, so daß die 2. Hülle vernetzt ist.

Als Mono-(meth)acrylate können beispielsweise verwendet werden : Methyl(meth)acrylat, Ethyl(meth)acrylat, Butyl (meth)acrylat, Lauryl(meth)acrylat, Cyclohexyl(meth)acrylat und Dihydrodicyclopentadienyl(meth)acrylat.

Als Di(meth)acrylate seien beispielhaft genannt : Ethylenglycoldi(meth)acrylat, Diethylenglycoldi(meth)acrylat, Triethylenglycoldi(meth)acrylat, Neopentylglycoldi(meth) acrylat, 1,12-Dodecandi(meth)acrylat, ferner Derivate des Bisphenol-A, wie 2,2-Bis- 4(2-hydroxy-3-methacryloyloxypropyl)-phenyl-propan (Bis GMA) sowie Urethandi(meth)acrylate, wie sie zum Beispiel in den US-PSen 34 25 988, 37 09 866 und 36 29 187 beschrieben sind.

Als Tri(meth)acrylate kommen z. B. in Frage : Glycerintri(meth)acrylat, Trimethylolpropantri(meth)acrylat und Pentaerythrittri(meth)acrylat.

Unter der Bezeichnung (Meth)acrylat sind sowohl das Methacrylat als auch das Acrylat gemeint. Zur Herstellung der erfindungsgemäßen Füllstoffe werden vorzugsweise die Methacrylate eingesetzt. Neben den (Meth) acrylaten können bis zu 20 Gew.-%, bezogen auf die Summe der (Meth)acrylatmonomeren, an weiteren Vinyl- bzw. Vinyliden-Monomeren, wie beispielsweise Styrol, α-Methylstyrol, Acrylnitril oder Vinylacetat eingesetzt werden. Die (Meth)acrylatmonomeren werden bevorzugt in Mengen von 3-100, besonders bevorzugt 10-50 Gew.-%, bezogen auf den hochdispersen Füllstoff, angewendet. Die Auswahl der angewendeten (Meth)acrylatmonomeren geschieht im Hinblick auf den gewünschten Einsatzzweck des erfindungsgemäßen Füllstoffes. So ist es z. B. zweckmäßig, für einen Einsatz in Kunststoffzähnen aus Polymethylmethacrylat zumindest anteilmäßig Methylmethacrylat in die 2. Hülle einzupolymerisieren, um eine gute Verträglichkeit zu erreichen.

Zur Einleitung der Polymerisation können übliche lösliche Radikalbildner verwendet werden : beispielhaft seien genannt : Peroxid- und Azoverbindungen, wie Dibenzoylperoxid, Dilauroylperoxid, Cyclohexylpercarbonat und Azoisobuttersäuredinitril. Gut geeignet sind auch Mischungen von Polymerisationsinitiatoren mit unterschiedlichen Zerfallstemperaturen. Die Polymerisation wird durch Erhitzen auf die Zerfallstemperatur des Polymerisationsinitiators eingeleitet. Die Polymerisation kann unter erhöhtem Druck, beispielsweise unter 2 bis 6 bar Stickstoffdruck, durchgeführt werden. Während der Polymerisation wird vorzugsweise mit einem hochtourigen Rührer gerührt.

Aus der auspolymerisierten Dispersion kann der erfindungsgemäße Füllstoff durch Abdampfen des Lösungsmittels gewonnen werden. Es ist auch möglich, das Polymerisat z. B. durch Zugabe von Methanol auszuflocken und zu filtrieren. Besonders vorteilhaft ist die Anwendung eines Sprühtrocknungsverfahrens, wobei ein feinteiliges, rieselfähiges Produkt erhalten wird.

Der erfindungsgemäße Füllstoff, insbesondere der durch Sprühtrocknung isolierte, läßt sich in vorteilhafter Weise in polymerisierbaren Massen, insbesondere bei der Herstellung von Dentalkunststoffen, einsetzen. Der Füllstoff ist gut benetzbar, hervorragend dispergierbar und läßt sich mit hohen Füllgraden in monomere Binder einarbeiten. Formkörper, z. B. künstliche Zähne, die den erfindungsgemäßen Füllstoff enthalten, zeichnen sich durch hohe mechanische Festigkeit und vor allem hohe Abrasionsresistenz aus.

Die erfindungsgemäßen polymerisierbaren Massen enthalten 20 bis 65 Gew.-%, bevorzugt 20 bis 50 Gew.-%, insbesondere 25 bis 45 Gew.-% eines polymerisierbaren Monomeren, 10 bis 75 Gew.-%, vorzugsweise 20 bis 75 Gew.-%, insbesondere 30 bis 70 Gew.-% des erfindungsgemäßen Füllstoffes sowie gegebenenfalls an sich bekannte weitere anorganische oder organische Füllstoffe, Polymerisationsinitiatoren, Inhibitoren, Farbstoffe und sonstige Zusatzstoffe. Bei Anwendung großer Anteile von mehrfunktionellen Monomeren (Vernetzern) und für bestimmte Einsatzzwecke kann es vorteilhaft sein, den erfindungsgemäßen polymerisierbaren Massen Weichmacher zur Verringerung der Sprödigkeit zuzusetzen. Gut geeignet sind in erster Linie an sich bekannte hochmolekulare Weichmacher, besonders solche auf Basis von Polyurethanen, Polycarbonaten, Polyestern und Polyethern. Bevorzugt werden Polyester und Polyestercarbonate, die in der DE-A-33 16 851 beschrieben werden. Als polymerisierbare Monomere und Polymerisationsinitiatoren kommen z. B. die oben im Zusammenhang mit der Herstellung der 2. Hülle des Füllstoffes genannten Verbindungen in Betracht. Die polymerisierbaren Massen eignen sich insbesondere für die Herstellung von Dentalformkörpern aber auch als Knochenzemente und für andere orthopädische Einsatzzwecke.

Beispiel 1

In einem heizbaren 40 l-Rührautoklaven werden 19,8 kg Methylenchlorid und 3,8 kg hochdisperses Siliziumdioxid (Teilchengröße ca. 20 nm, BET-Oberfläche 50 m²/g) eingewogen und die Rührerdrehzahl auf 400 UpM eingestellt. In einem gesonderten Glasgefäß werden 760 g γ-Methacryl-oxypropyl-trimethoxysilan, 980 g entionisiertes Wasser und 10,4 g Methacrylsäure gemischt. Sobald die Mischung einphasig ist (nach ca. 40 Min. bei Raumtemperatur), wird sie in einer Portion in den Rührautoklaven gegeben. Die Temperatur im Rührautoklaven wird 20 Stunden auf 40 °C gehalten, danach werden 0,38 kg Methylmethacrylat, 0,38 kg Isobutylenmethacrylat, 8 g Ethylendimethacrylat und 20 g Azoisobuttersäuredinitril zugesetzt, ein Druck von 5 bar N angelegt, und die Temperatur 3 Stunden auf 70 °C und anschließend 3 weitere Stunden auf 80 °C gehalten. Nach dem Abkühlen wird die Dispersion mit 10 kg Methylenchlorid verdünnt und einem Sprühtrocknungsprozeß unterzogen. Man erhält 5,1 kg feinteiligen Füllstoff.

Beispiel 2

Beispiel 1 wird wiederholt, wobei folgende Einsatzmengen angewendet werden : 19,8 kg Methylenchlorid, 3,8 kg hochdisperses Siliziumdioxid (Teilchengröße ca. 20 nm, BET.: Oberfläche 50 m²/g), 456 g γ-Methacryl-oxypropyl-trimethoxysilan, 588 g entionisiertes Wasser, 6 g Methacrylsäure, 550 g Methylmethacrylat, 11 g Ethylendimethacrylat und 20 g Azoisobuttersäuredinitril. Man erhält 4,8 kg feinteiligen Füllstoff.

Beispiel 3

In einem Kneter werden 65 g Bisphenol-A-diglycidyldimethacrylat, 35 g Triethylenglycoldimethacrylat, 318 g des feinteiligen Füllstoffes aus Beispiel 1, 2 g Dibenzoylperoxid und geringe Mengen (< 0,1 g) übliche Pigmente zu einer Paste geknetet. Zur Herstellung von Zähnen wird diese Paste in Formen gepreßt und bei 130 °C in 6 Minuten gehärtet. Die erhaltenen Zähne zeichnen sich durch große Härte und hohe Abrasionsfestigkeit aus.

Beispiel 4

In einem Kneter werden 75 g Methylmethacrylat, 20 g Ethylendimethacrylat, 5 g eines Polycarbonatgruppenenthaltenden Polyesters (Beispiel 3 der DE-OS 2 732 718), 20 g Dibenzoylperoxid, 30 g Polymethyl-methacrylatperlpolymerisat (d₅₀ : 45 μm, [η] in CHCl₃ : 1,8 dl/g) und 220 g feinteiliger Füllstoff aus Beispiel 2 zu einer pastösen Masse geknetet. Diese Masse wird mit 2 g Dibenzoylperoxid aktiviert und mit üblichen Pigmenten zahnfarbig eingefärbt. Nach einer Reifezeit von 30 Min. wird die Masse in Zahnformen gepreßt und bei 120 °C 8 Minuten ausgehärtet. Die erhaltenen Zähne zeichnen sich durch große Härte und hohe Abrasionsfestigkeit aus.

**Patentansprüche**

1. Füllstoff für polymerisierbare Massen mit einem anorganischen Kern aus Siliziumdioxid, Silikaten oder Aluminiumoxid, der eine Teilchengröße von 10 bis 500 nm aufweist, und einer Hülle aus polymerisiertem Vinylsilan, wobei der Anteil des Vinylsilans 2 bis 40 Gew.-%, bezogen auf den anorganischen Kern, beträgt, dadurch gekennzeichnet, daß der Füllstoff eine zweite Hülle aus (Meth)acrylatpolymeren aufweist.

2. Füllstoff nach Anspruch 1, dadurch gekennzeichnet, daß die zweite Hülle 3 bis 100 Gew.-% (Meth)acrylat, bezogen auf den anorganischen Kern, enthält.

3. Füllstoff nach den Ansprüchen 1 und 2, dadurch gekennzeichnet, daß die zweite Hülle aus (Meth)acrylat vernetzt ist.

4. Verfahren zur Herstellung eines Füllstoffs für polymerisierbare Massen mit einem anorganischen Kern mit einer Teilchengröße von 10 bis 500 nm, einer ersten Hülle aus polymerisiertem Vinylsilan, wobei der Anteil des Vinylsilans 2 bis 40 Gew.-%, bezogen auf den anorganischen Kern, beträgt, und einer zweiten Hülle aus (Meth)acrylatpolymeren, dadurch gekennzeichnet, daß man für die erste Hülle zuerst die Silanverbindung durch Umsetzung mit etwa gleichen Gewichtsteilen Wasser in Gegenwart von 0,1 bis 5 Gew.-% einer Säure in eine aktivierte Form überführt, dann den anorganischen Kern in einer Konzentration von 5 bis 40 Gew.-% in einem organischen Lösungsmittel dispergiert und im Temperaturbereich von 0 bis 100 °C mit dem aktivierten Silan-Wasser-Gemisch versetzt, und dann für die zweite Hülle den silanisierten anorganischen Kern in einem inerten organischen Lösungsmittel mit (Meth)acrylatmonomeren und mit einem Polymerisationsstarter versetzt und die Polymerisationsreaktion durchführt.

5. Verfahren nach Anspruch 4, dadurch gekennzeichnet, daß der Füllstoff durch ein Sprühtrockungsverfahren aus dem Lösungsmittel isoliert wird.

6. Dentalkunststoffe, enthaltend einen Füllstoff für polymerisierbare Massen mit einem anorganischen Kern mit einer Teilchengröße von 10 bis 500 nm, einer ersten Hülle aus polymerisiertem Vinylsilan, wobei der Anteil des Vinylsilans 2 bis 40 Gew.-%, bezogen auf den anorganischen Kern, beträgt und einer zweiten Hülle aus (Meth)acrylatpolymeren.

7. Verwendung von Füllstoffen für polymerisierbare Massen mit einem anorganischen Kern mit einer Teilchengröße von 10 bis 500 nm, einer ersten Hülle aus polymerisiertem Vinylsilan, wobei der Anteil des Vinylsilans 2 bis 40 Gew.-%, bezogen auf den anorganischen Kern, beträgt und einer zweiten Hülle aus (Meth)acrylatpolymeren in Dentalkunststoffen.

8. Verwendung nach Anspruch 7 für künstliche Zähne.

## Claims

1. Filler for polymerisable compositions having an inorganic core of silicon dioxide, silicates or aluminium oxide, which has a particle size of 10 to 500 nm, and a shell of polymerised vinylsilane, the proportion of the vinylsilane being 2 to 40 % by weight based on the inorganic core, characterised in that the filler has a second shell of (meth)acrylate polymers.

2. Filler according to Claim 1, characterised in that the second shell contains 3 to 100 % by weight of (meth)acrylate, based on the inorganic core.

3. Filler according to Claims 1 and 2, characterised in that the second shell of (meth)acrylate is crosslinked.

4. Process for the preparation of a filler for polymerisable compositions having an inorganic core with a particle size of 10 to 500 nm, a first shell of polymerised vinylsilane, the proportion of the vinylsilane being 2 to 40 % by weight, based on the inorganic core, and a second shell of (meth)acrylate polymers, characterised in that, for the first shell, the silane compound is first converted into an activated form by reacting it with approximately equal parts by weight of water in the presence of 0.1 to 5 % by weight of an acid, then the inorganic core is dispersed in an organic solvent in a concentration of 5 to 40 % by weight and the activated silane/water mixture is added at a temperature in the range 0 to 100 °C, and then, for the second shell, (meth)acrylate monomers and a polymerisation initiator are added to the silanised inorganic core in an inert organic solvent and the polymerisation reaction is carried out.

5. Process according to Claim 4, characterised in that the filler is isolated from the solvent by a spray-drying process.

6. Dental plastics containing a filler for polymerisable compositions having an inorganic core with a particle size of 10 to 500 nm, a first shell of polymerised vinylsilane, the proportion of the vinylsilane being 2 to 40 % by weight, based on the inorganic core, and a second shell of (meth)acrylate polymers.

7. Use of fillers for polymerisable compositions having an inorganic core with a particle size of 10 to 500 nm, a first shell of polymerised vinylsilane, the proportion of the vinylsilane being 2 to 40 % by weight, based on the inorganic core, and a second shell of (meth)acrylate polymers in dental plastics.

8. Use according to Claim 7 for artificial teeth.

## Revendications

1. Matière de charge pour masses polymérisables, avec noyau minéral en dioxyde de silicium, en silicates ou en oxyde d'aluminium, qui présente une dimension de particule de 10 à 500 nm, et avec enveloppe en vinylsilane polymérisé, la proportion de vinylsilane s'élevant à 2 à 40 % en poids par rapport au noyau minéral, caractérisée en ce que la matière de charge présente une deuxième enveloppe en polymères de (méth)acrylates.

2. Matière de charge selon la revendication 1,

caractérisée en ce que la deuxième enveloppe contient 3 à 100 % en poids de (méth)acrylate par rapport au noyau minéral.

3. Matière de charge selon les revendications 1 et 2, caractérisée en ce que la deuxième enveloppe en (meth)acrylate est réticulée.

4. Procédé de fabrication d'une matière de charge pour masses polymérisables, ayant un noyau minéral présentant une dimension de particule de 10 à 500 nm, une première enveloppe en vinylsilane polymérisé, la proportion du vinylsilane s'élevant à 2 à 40 % en poids par rapport au noyau minéral, et une deuxième enveloppe en polymères de (méth)acrylates, caractérisé en ce que pour la première enveloppe on convertit d'abord le composé silanique par réaction avec des parties en poids d'eau sensiblement égales en présence de 0,1 à 5% en poids d'un acide en une forme activée, on disperse alors le noyau minéral à une concentration de 5 à 40% en poids dans un solvant organique et l'on y ajoute dans l'intervalle de température de 0 à 100 °C le mélange activé silane-eau, on ajoute ensuite pour la deuxième enveloppe au noyau minéral silanisé dans un solvant organique inerte les monomères de (méth)acrylates et un initiateur de polymérisation et l'on exécute la réaction de polymérisation.

5. Procédé selon la revendication 4, caractérisé en ce qu'on isole la matière de charge d'avec le solvant par un procédé de séchage par pulvérisation.

6. Matières plastiques dentaires, contenant une matière de charge pour masses polymérisables qui comporte un noyau minéral d'une dimension de particule de 10 à 500 nm, une première enveloppe en vinylsilane polymérisé, la proportion de vinylsilane s'élevant à 2 à 40 % en poids par rapport au noyau minéral, et une deuxième enveloppe en polymères de (méth)acrylates.

7. Utilisation de matières de charge pour masses polymérisables qui comportent un noyau minéral d'une dimension de particule de 10 à 500 nm, une première enveloppe en vinylsilane polymérisé, la proportion du vinylsilane s'élevant à 2 à 40 % en poids par rapport au noyau minéral, et une deuxième enveloppe en polymères de (méth)acrylates, dans des matières plastiques dentaires.

8. Utilisation selon la revendication 7 pour des dents artificielles.